# FASCICULE DE BREVET EUROPEEN

(11) **EP 1 651 188 B1**
(45) Date de publication et mention de la délivrance du brevet: **06.05.2015**
(21) Numéro de dépôt: 04767702.6
(22) Date de dépôt: 16.07.2004
(51) Int. Cl.: A61K 9/14

(54) **PARTICULES COMPRENANT UN PRINCIPE ACTIF SOUS FORME DE CO-PRECIPITE**
EINEN WIRKSTOFF IN FORM EINES CO-PRÄZIPITATS ENTHALTENDE TEILCHEN
PARTICLES CONTAINING AN ACTIVE AGENT IN THE FORM OF A CO-PRECIPITATE

(30) Priorité: 17.07.2003 FR 0308720
(43) Date de publication de la demande: 03.05.2006
(73) Titulaire: ETHYPHARM, 78550 Houdan (FR)
(72) Inventeur: COUSIN, Gérard, F-28210 Villemeux sur Eure (FR); LAMOUREUX, Gaël, F-28210 Le Boullay Thierry (FR)
(74) Mandataire: Regimbeau
(86) Numéro de dépôt international: PCT/FR2004/001878
(87) Numéro de publication internationale: WO 2005/009411

(56) Documents cités:
- EP-A- 1 103 252
- FR-A- 2 803 748

## Description

L'invention concerne des particules comprenant une substance active sous la forme d'un co-précipité, le procédé de préparation desdites particules et les formes pharmaceutiques comprenant ces particules.

La préparation de médicament par voie orale se heurte à des limites avec des molécules de synthèse de plus en plus complexes présentant souvent des caractéristiques physico-chimiques défavorables à l'absorption de la molécule au niveau du tractus gastro-intestinal, et en particulier une très faible solubilité en milieux aqueux.

Cela se traduit souvent par une biodisponibilité très faible de ces molécules, et la nécessité d'administrer aux patients des doses élevées de substance active pour atteindre la concentration efficace.

Ceci est d'autant plus préjudiciable quand la concentration efficace à atteindre est élevée, que le nombre de comprimés ou de gélules à prendre en une prise est important, ou que le nombre de prises par jour est plus fréquent.

La préparation de dispersions solides est une des méthodes qui permettent d'améliorer sensiblement la solubilité des principes actifs, et par là, leur biodisponibilité.

Les dispersions solides sont habituellement obtenues selon deux méthodes, d'une part en évaporant une solution comprenant la substance active et le support inerte, d'autre part, par fusion des deux composés précédents, puis solidification.

Dans le premier cas, le produit est un co-précipité, dans le second cas, c'est un co-fondu.

Une première difficulté est d'obtenir des particules dont la vitesse de solubilisation est la plus rapide possible et la fraction solubilisée, la plus élevée possible, pour assurer une biodisponibilité suffisante de la molécule.

Une seconde difficulté est d'obtenir des particules ayant une distribution granulométrique étroite, et présentant une taille compatible avec une mise en forme destinée à une administration par voie orale, tout en conservant une teneur élevée en substance active.

Une troisième difficulté est d'obtenir une formulation qui soit stable dans le temps dans des conditions de température et d'humidité défavorables, telle que celles utilisées dans les études de stabilité, par exemple 30°C/60% HR (humidité résiduelle), ou 40°C/75% HR.

Les dispersions solides sont en général constituées d'une substance active dispersée à l'état moléculaire dans un agent hydrophile, par exemple un polymère.

La demande internationale WO 97/04749 a pour objet un procédé de préparation de dispersions solides dans lequel la ou les substances active(s) est (sont) dissoute(s) dans un solvant organique contenant en outre une amide cyclique très hydrophile et de façon avantageuse un agent tensioactif, la solution organique ainsi obtenue étant ensuite évaporée à sec, puis broyée et tamisée. L'amide cyclique est une polyvinylpyrrolidone dont le poids moléculaire varie de 10 000 à 50 000.

La demande EP 1 103 252 décrit des compositions pharmaceutiques comprenant des particules ayant un coeur recouvert d'une couche unique incluant un principe actif antifongique comme l'itraconazole, ladite couche étant obtenue par pulvérisation d'une solution comprenant le principe actif, un polymère hydrophile et un tensioactif non ionique. Ces compositions, obtenues par des techniques classiques, améliorent la solubilité du principe actif.

Dans les cas extrêmes, l'obtention d'une dispersion solide n'est en elle-même pas suffisante pour améliorer de façon significative la biodisponibilité.

La Demanderesse a montré qu'il est tout à fait possible d'améliorer de façon substantielle la biodisponibilité de substances actives pratiquement insolubles dans l'eau, et d'en augmenter la vitesse de solubilisation et la fraction solubilisée *in vitro* ou *in vivo,* en préparant une particule constituée d'un co-précipité appliqué en couche autour d'un support neutre hydrophile et comprenant au moins, une substance active, un agent tensioactif, et un polymère hydrophile, avec le procédé de la revendication 1.

La couche formée autour du support est une dispersion solide sous la forme d'un co-précipité, facilement soluble dans l'eau ou les milieux physiologiques, par exemple le liquide gastrique.

Par ailleurs, la Demanderesse a montré qu'il est tout à fait possible d'améliorer de façon substantielle les problèmes d'agglomération de particules, rencontrés dans les cas de pulvérisation de grandes quantités solutions d'enrobage, en broyant les particules, non pas uniquement à la fin de l'étape de pulvérisation, mais au cours de cette étape.

La réduction de taille consécutive au broyage permet alors de poursuivre la pulvérisation sur des particules de plus petites tailles, moins aptes à s'agglomérer entre elles que des particules dont la croissance est régulière au cours d'une étape unique de pulvérisation.

Le co-précipité est obtenu par pulvérisation d'une solution organique sur un support neutre hydrophile, ladite solution comprenant au moins une substance active, un agent tensioactif et un polymère hydrophile, ladite pulvérisation étant caractérisée en ce que la pulvérisation de la totalité de la solution est réalisée en au moins deux étapes distinctes, chacune de ces étapes étant systématiquement suivie d'une étape de broyage du produit obtenu à l'issue de l'étape précédente.

Le procédé de préparation des particules comprend les étapes suivantes :
a) préparation d'une solution organique comprenant la substance active, le polymère hydrophile et l'agent tensioactif,
b) pulvérisation d'une partie de la solution obtenue en a) sur les supports neutres hydrophiles,
c) broyage des particules obtenues à l'étape b),
d) pulvérisation de la quantité restante de la solution organique sur les particules broyées à l'étape c), et
e) broyage final des particules obtenues à l'étape d).

La séquence pulvérisation/broyage (étapes b à d) peut être répétée une ou plusieurs fois selon le volume de solution à pulvériser et la cinétique de croissance des particules au cours de l'étape de pulvérisation.

La totalité de la solution organique peut être préparée en une fois; cependant pour éviter une évaporation trop importante des solvants organiques, il est préférable de préparer chaque fraction de la solution juste avant l'étape de pulvérisation.

La pulvérisation de la solution organique peut être réalisée dans une turbine à dragéification, une turbine perforée ou en lit fluidisé.

Dans un mode de réalisation préféré du procédé conforme à l'invention, toutes les étapes de pulvérisation sont effectuées en lit fluidisé, équipé d'un dispositif antidéflagrant.

Le lit fluidisé est équipé d'une buse de pulvérisation dont la position et l'orientation de pulvérisation peuvent être choisies.

Ce choix permet de maîtriser la cinétique de croissance des particules et d'éviter des phénomènes de collage, liés à la nature de la substance active, à la composition qualitative et quantitative de la solution enrobante pulvérisée, et aux divers paramètres du procédé (par exemple la température, la pression d'air et le débit de pulvérisation).

Un séchage des particules est habituellement réalisé après la pulvérisation de la solution organique.

Le séchage peut être réalisé sur des plateaux ou directement dans l'équipement utilisé pour l'étape de pulvérisation.

Le séchage peut être réalisé, soit juste après la pulvérisation de la solution organique et avant le broyage, soit immédiatement après le broyage des particules.

Le broyage peut être réalisé sur tout type d'équipement destiné à cet effet, et peut être un broyeur de type oscillant ou équipé de broches.

Le broyeur rotatif de type FITZMILL ou le broyeur oscillant de type FREWITT, est équipé d'un rotor qui force les particules à travers une grille d'ouvertures calibrées.

Le broyeur de type FORPLEX est équipé de broches sur lesquelles les particules sont lancées à grande vitesse.

Le broyeur utilisé entre chaque étape de pulvérisation peut être différent de celui utilisé pour le broyage final.

Le procédé de l'invention est particulièrement adapté pour les substances actives très peu solubles dans l'eau, c'est à dire les substances actives dont une partie est soluble dans 1 000 parties d'eau ou plus de 1 000 parties d'eau, de préférence pour les substances actives pratiquement insolubles dans l'eau, c'est à dire celles dont une partie est soluble dans 10 000 parties d'eau ou plus de 10 000 parties d'eau.

La ou les substances actives peuvent être choisies dans toute famille de composés, par exemple parmi les sédatifs gastro-intestinaux, les antiacides, les antalgiques, les anti-inflammatoires, les vasodilatateurs coronariens, les vasodilatateurs périphériques et cérébraux, les anti-infectieux, les antibiotiques, les antiviraux, les antiparasitaires, les anticancéreux, les anxiolytiques, les neuroleptiques, les stimulants du système nerveux central, les antidépresseurs, les antihistaminiques, les anti-diarrhéiques, les laxatifs, les suppléments nutritionnels, les immunodépresseurs, les hypocholestérolémiants, les hormones, les enzymes, les antispasmodiques, les anti-angoreux, les médicaments influençant le rythme cardiaque, les médicaments utilisés dans le traitement de l'hypertension artérielle, les antimigraineux, les médicaments influençant la coagulabilité sanguine, les antiépileptiques, les myorelaxants, les médicaments utilisés dans le traitement du diabète, les médicaments utilisés dans le traitement des dysfonctions thyroïdiennes, les diurétiques, les anorexigènes, les antiasthmatiques, les expectorants, les antitussifs, les mucorégulateurs, les décongestionnants, les hypnotiques, les antinauséeux, les hématopoïétiques, les uricosuriques, les extraits de végétaux, les agents de contraste ou tout autre famille de composés, les substances actives associées dans le comprimé pouvant être choisies dans la même famille ou dans des familles différentes.

Les substances actives peuvent se présenter sous la forme de leurs sels pharmaceutiquement acceptables ou toute forme polymorphe (racémique, énantiomère,...). Par "sels pharmaceutiquement acceptables", on entend les dérivés des composés décrits dans lesquels le composé pharmaceutiquement actif de base est transformé en son sel basique ou acide, des exemples de sels pharmaceutiquement actifs comprennent notamment les sels d'acides organiques ou minéraux de résidus basiques tels que les amines; les dérivés alcalins ou les sels organiques de résidus acides tels que les acides carboxyliques, et similaires.

La substance active est présente dans la particule dans une proportion pouvant varier entre 1 et 60% en poids.

Le support inerte hydrophile peut être constitué par tout excipient inerte chimiquement et pharmaceutiquement, existant sous forme particulaire, cristalline ou amorphe, par exemple des dérivés de sucres tels que le lactose, de préférence le lactose Extra Fin (EFK), le saccharose, l'amidon hydrolysé (maltodextrines); des celluloses ou des mélanges tels que le saccharose et l'amidon, ou des mélanges à base de cellulose peuvent également être utilisés pour la préparation de supports inertes sphériques.

Le support inerte hydrophile est présent dans une proportion pouvant aller jusqu'à 95% en poids.

La dimension particulaire unitaire du support inerte hydrophile peut être comprise entre 50 et 500 µm, de préférence entre 90 et 200 µm.

Le polymère hydrophile peut être choisi parmi les polyvinylpyrrolidones et les dérivés cellulosiques, les polymères acryliques et les polyéthylènes glycols.

La polyvinylpyrrolidone peut être choisie parmi les polymères de poids moléculaire compris entre 10 000 et 50 000.

Le dérivé cellulosique est choisi parmi les dérivés hydroxylés, par exemple l'hydroxypropylméthylcellulose, l'hydroxypropylcellulose, l'hydroxyméthyl-cellulose, l'hydroxypropylméthylcellulose phtalate et l'hydroxypropyl méthylcellulose acéto-succinate.

L'hydroxypropylméthylcellulose préférée est choisie parmi celles dont la viscosité apparente est comprise entre 2,4 et 18 cP, et de manière encore plus préférée, comprise entre 2,4 et 5cP.

Le polymère acrylique peut être choisi parmi le copolymère ammoniométhacrylate, le polyacrylate, le polyméthacrylate et le copolymère d'acide méthacrylique.

Le polyéthylène glycol peut être choisi parmi les polymères de poids moléculaire compris entre 1 000 et 20 000.

Le rapport pondéral polymère hydrophile/principe actif est compris de préférence entre 10/1 et 1/2.

L'agent tensioactif peut être choisi parmi les agents cationiques, anioniques, non ioniques ou amphotères, seuls ou en mélange.

L'agent tensioactif peut être choisi par exemple parmi les composés tels que le laurylsulfate de sodium, le monooléate, le monolaurate, le monopalmitate, le monostéarate, le trioléate, le tristéarate ou tout autre ester de sorbitanne polyoxyéthyléné, de préférence les Tween^{®} 20, 40, 60 ou 80, des glycérides d'acides gras polyoxyéthylénés, ces acides gras étant saturés ou non saturés, et constitués d'au moins 8 atomes de carbones, des poloxamères tel que le poloxamère 188, des copolymères blocs oxyde d'éthylène/oxyde de propylène tels que les Pluronic^{®} F68 ou F87, la lécithine, l'alcool stéarylique, l'alcool cétostéarylique, la lécithine, le cholestérol, l'huile de ricin polyoxyéthylénée, les éthers polyoxyéthylénés d'alcool gras, comme les Brij^{®} et les stéarates polyoxyéthylénés.

L'agent tensioactif est présent avantageusement dans une proportion pouvant varier entre 0,1 et 20% en poids par rapport à la masse totale obtenue.

Le solvant organique peut être choisi parmi l'éthanol, l'isopropanol, le tétrahydrofuranne, l'éther isopropylique, l'acétone, la méthyléthylacétone, le chlorure de méthylène ou un mélange de ces solvants.

Le volume de solvant tient compte de la solubilité des différents composants de la solution organique.

La présente invention a également pour objet une particule constituée d'un co-précipité appliqué en couche autour d'un support et comprenant au moins, une substance active, un agent tensioactif et un polymère hydrophile, susceptible d'être obtenue par pulvérisation d'une solution comprenant au moins une substance active, un agent tensioactif, et un polymère hydrophile, ladite pulvérisation étant réalisée au moins en deux étapes distinctes, lesdites étapes étant chacune suivie d'une étape de broyage.

Il résulte du procédé de l'invention que, si le support sur lequel est pulvérisée la solution comprenant au moins une substance active, un agent tensioactif, et un polymère hydrophile est, à la première étape, bien dépourvu de toute substance active, le support résultant du premier broyage, sur lequel est pulvérisée la même solution, est constitué à la fois du support neutre hydrophile, et du co-précipité comprenant au moins, une substance active, un agent tensioactif et un polymère hydrophile.

La teneur en principe actif du support utilisé pour appliquer la solution comprenant au moins une substance active, un agent tensioactif, et un polymère hydrophile augmente donc après chaque cycle pulvérisation/broyage.

Après la dernière étape de pulvérisation, la structure de la particules est une sphère constituée d'une coeur comprenant un mélange de support neutre hydrophile et de co-précipité comprenant au moins une substance active, un agent tensioactif, et un polymère hydrophile, et d'une couche externe du même mélange comprenant au moins une substance active, un agent tensioactif, et un polymère hydrophile.

La taille des particules est habituellement comprise entre 50 et 1000 µm, de préférence entre 100 et 800 µm, de façon encore préférée entre 200 et 500 µm, et est déterminée par les méthodes conventionnelles, par exemple à l'aide d'un jeu de tamis d'ouverture de mailles calibrées, ou par diffraction d'un laser.

Dans une mise en oeuvre préférée de l'invention, les particules de l'invention contiennent:
- de 15 à 40% en poids d'un support inerte hydrophile, de préférence le lactose EFK
- de 15 à 30% en poids de substance active
- 30 à 60% en poids de polymère hydrophile, de préférence l'HPMC
- de 1 à 10% en poids d'agent tensioactif, de préférence un agent non ionique choisi dans le groupe comprenant les polysorbates 20 à 80.

Les particules selon l'invention peuvent être utilisées directement ou mélangées à des excipients utilisés dans le domaine pharmaceutique pour la préparation d'une forme pharmaceutique destinée à être administrée par voie orale, telle que par exemple une gélule ou un comprimé et compatibles chimiquement avec la ou les substances actives.

Ces excipients peuvent être choisis parmi les diluants, les agents de désintégration, les agents gonflants, les lubrifiants, les agents antistatiques, les liants ou des adjuvants.

Le diluant peut être choisi parmi les sucres tels que le saccharose, le lactose, le fructose, le dextrose, ou les polyols de moins de 13 atomes de carbone tels que le mannitol, le xylitol, le sorbitol, le maltitol, le lactitol, l'érythritol, le phosphate dicalcique, le phosphate tricalcique ou une cellulose microcristalline, seuls ou en mélange.

Le diluant est utilisé dans une proportion comprise entre 20 et 90 % en poids, de préférence entre 30 et 60% en poids, calculée par rapport au poids du comprimé.

Le diluant est de préférence utilisé sous sa forme directement compressible, dont le diamètre moyen des particules est de 100 à 500 µm, ou sous la forme d'une poudre dont le diamètre moyen des particules est inférieur à 100 µm, ladite poudre étant utilisée seule ou en mélange avec le produit directement compressible.

L'agent de désintégration est choisi dans le groupe comprenant notamment la carboxyméthylcellulose sodique réticulée désignée par le terme croscarmellose, les polyvinylpyrrolidones réticulées désignées par le terme crospovidone, et leurs mélanges.

L'agent de désintégration est utilisé dans une proportion comprise entre 1 et 20% en poids, de préférence entre 5 et 15% en poids, calculée par rapport au poids du comprimé.

L'agent gonflant est choisi dans le groupe comprenant la cellulose microcristalline, les amidons, les amidons modifiés, tels que le carboxyméthylamidon ou le glycolateamidon sodique, l'acide alginique ou l'alginate de sodium, et leurs mélanges.

L'agent gonflant est utilisé dans une proportion comprise entre 1 et 15% en poids calculée par rapport au poids du comprimé.

Le lubrifiant est choisi dans le groupe comprenant le stéarate de magnésium, l'acide stéarique, le sodium stéaryl fumarate, les polyoxyéthylèneglycols, le benzoate de sodium, une huile pharmaceutiquement acceptable, de préférence la diméthicone ou la paraffine liquide et leurs mélanges.

Le lubrifiant est utilisé dans une proportion pouvant aller jusqu'à 2%, de préférence entre 0,02 et 2% en poids, de façon encore préférée entre 0,5 et 1% en poids, calculée par rapport au poids du comprimé.

Selon une première variante, le lubrifiant est incorporé en totalité au mélange d'excipients de compression; dans une seconde variante, une fraction de ce lubrifiant est pulvérisée sur les parois de la matrice et des poinçons au moment de la compression, le lubrifiant étant alors sous la forme d'une poudre, par exemple le stéarate ou magnésium ou d'un liquide, par exemple l'huile de paraffine liquide.

Les quantités de lubrifiant utilisées en phase interne et/ou externe sont ajustées avec soin de façon à éviter qu'un excès n'altère la cohésion du lit de poudre lors de la compression.

L'agent antistatique peut être choisi dans le groupe comprenant le talc micronisé ou non micronisé, la silice colloïdale (Aerosil^{®}200), la silice traitée (Aerosil^{®}R972), ou la silice précipitée (Syloïd^{®} FP244) et leurs mélanges.

L'agent antistatique est utilisé dans une proportion pouvant aller jusqu'à 5% en poids, calculée par rapport au poids du comprimé.

Le liant est utilisé sous forme sèche et peut être un amidon, un sucre, la polyvinylpyrrolidone ou la carboxyméthylcellulose, seuls ou en mélange.

Le liant est utilisé dans une proportion pouvant aller jusqu'à 15% en poids, de préférence inférieure à 10% en poids, calculée par rapport au poids du comprimé.

Des adjuvants peuvent également être ajoutés au mélange destiné à être mis en gélules ou à être comprimé et sont choisis dans le groupe comprenant les agents ajusteurs de pH, les systèmes permettant de produire une effervescence, notamment générateurs de dioxyde de carbone du type de ceux utilisés comme agents ajusteurs de pH, ou encore les tensioactifs.

L'invention a également pour objet des formes pharmaceutiques comprenant les particules conformes à l'invention.

La préparation de la forme pharmaceutique comprenant les particules de l'invention peut comprendre les étapes suivantes :
- mélange sec des particules, obtenues selon le procédé décrit précédemment, avec des excipients.
- compression du mélange ou mise en gélule du mélange pour obtenir une forme unitaire.

Dans un mode avantageux de mise en oeuvre la totalité du lubrifiant est pulvérisée sur les poinçons et /ou sur la face interne des matrices de compression la deuxième étape du mélange est alors bien entendu supprimée.

Dans un autre mode avantageux de préparation de la forme pharmaceutique, la préparation du mélange comprend deux étapes, la première étape consistant à mélanger la substance active avec tous les excipients de compression sauf le lubrifiant interne, puis une seconde étape, dans laquelle le lubrifiant est ajouté au premier mélange en totalité ou en partie, la partie restante étant alors pulvérisée sur les poinçons et /ou sur la face interne des matrices de compression.

La compression du mélange peut être réalisée sur une machine à comprimer alternative ou rotative.

La dureté du comprimé est adaptée pour permettre d'obtenir une friabilité, mesurée selon la méthode de la Pharmacopée Européenne, inférieure à 2%, de préférence 1%.

Les comprimés peuvent être de forme ronde, ovale, oblongue, présenter une surface plate, concave ou convexe, et éventuellement présenter des gravures ou être chanfreinés.

Les comprimés ont de façon générale une masse comprise entre 0,1 gramme et 2,0 grammes et une taille de diamètre compris entre 6 mm et 18 mm.

L'exemple et les figures 1 à 4 qui suivent illustrent l'invention.
La figure 1 illustre la distribution granulométrique, étudiée en granulométrie laser, des particules préparées selon le procédé de l'invention.
La figure 2 représente la cinétique de dissolution de l'itraconazole seul (▲), sous la forme de particules préparées selon le procédé de l'invention avant broyage final (◊) et après broyage final (■).
La figure 3 illustre la distribution granulométrique, étudiée en granulométrie laser, des particules sans tensioactif.
La figure 4 illustre la cinétique de dissolution de l'itraconazole avant broyage avec (■) et sans (◆) tensioactif et après broyage avec (X) et sans (▲) tensioactif.

### Exemple 1

### 1. Préparation des particules contenant un tensioactif

La fabrication est réalisée sur le lit fluidisé GPCG1 en mode Bottom Spray.

La solution de pulvérisation est préparée par solubilisation de l'itraconazole (fourni par WICKOFF) dans un mélange de solvants alcool 96°/chlorure de méthylène dans un rapport pondéral 41,6/58,4 avec de l'HPMC 2910 5 cps (fournie par Dow Chemical) et du polysorbate 20 (Montanox® 20, fourni par SEPPIC).

Le lactose EFK (fourni par HMS) est introduit dans le lit fluidisé et la solution est pulvérisée en mode Bottom spray; 4 étapes de pulvérisation de la solution sont réalisées successivement; les granulés sont séchés en lit fluidisé et broyés à l'aide d'un broyeur Forplex avec une grille de 630 µm pour la présente utilisation après chaque étape de pulvérisation.

Après la quatrième étape, les granulés obtenus sont séchés dans le lit fluidisé. Les paramètres de montage sont présentés dans le tableau 1:

**Tableau 1**

| | Montages | | | |
|---|---|---|---|---|
| | 1 ère étape | 2ème étape | 3ème étape | 4ème étape |
| Débit de pulvérisation | 45 g/min | 39 g/min | 39 g/min | 39 g/min |
| Pression de pulvérisation | 0, 15 MPa | 0,15 MPa | 0,15 MPa | 0,15 MPa |
| Quantité de solution pulvérisée | 2,7 kg | 3,1 kg | 2,7 kg | 2,5 kg |
| Consigne air d'entrée | 50 - 60°C | 48 - 56°C | 56 - 58°C | 52 - 54°C |
| Température d'air d'entrée | 42 - 50°C | 50 - 56°C | 55 - 59°C | 51 - 55°C |
| Température du produit | 29 - 33°C | 27 - 34°C | 27 - 34°C | 28 - 33°C |

La masse totale de solution pulvérisée est de 11kg et le temps total de granulation est de 4h47 min.

Les particules obtenues sont séchées en lit fluidisé GPCG1, les paramètres de séchage étant les suivants:
- température d'air d'entrée : 48°C,
- temps de séchage : 30 min,
- refroidissement jusqu'à ce que la température atteigne 29°C.

Les particules sont ensuite broyées à l'aide d'un broyeur de type Forplex équipé d'une grille d'ouverture 630 µm.

La quantité de granulés secs obtenus est de 1,406 kg, correspondant à un rendement de 93%.

La formule de fabrication testée est indiquée dans le tableau 2.

**Tableau 2**

| Matières premières | Quantité (g) | % |
|---|---|---|
| Lactose EFK | 600 | 39,7 |
| Itraconazole | 350 | 23,2 |
| HMPC 2910 5 cps | 525 | 34,8 |
| Polysorbate 20 | 35 | 2,3 |
| Alcool 96° | 4200 | / |
| Chlorure de méthylène | 5900 | / |
| Quantité de solution pulvérisée | 11010 | / |
| Masse sèche théorique | 1510 | / |
| Titre théorique | 231,79 mg/g | / |

### 2. Résultats

### 2.1. Distribution granulométrique

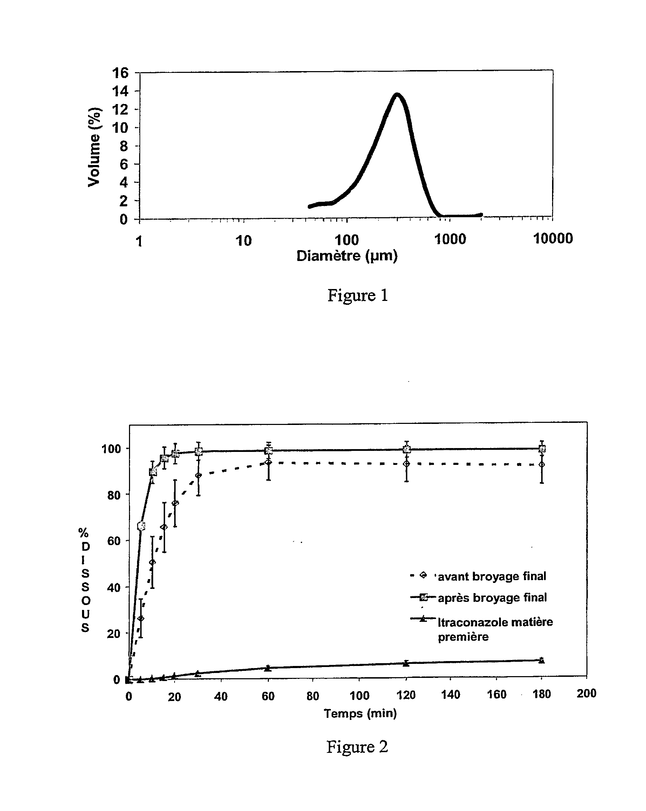

Elle est présentée dans la figure 1. La distribution granulométrique donne les résultats suivants:
- D_{10%} :62 µm
- D_{50%} : 231 µm
- D_{90%} : 419 µm.

### 2.2. Cinétiques de dissolution

Elles sont étudiées sur trois vases.

Les mesures sont faites en UV continu, à une longueur d'onde de mesure de 254 nm, sur 50 mg d'itraconazole dans 900 ml de milieu 1,2 type D (vitesse des pales 100 rpm).

Les résultats sont illustrés dans la figure 2.

Le montage réalisé selon le procédé de l'invention améliore considérablement la dissolution de l'itraconazole. Le broyage accélère la dissolution : 50% du produit est dissous au bout de 10 minutes d'essai pour la fraction non broyée, et au bout de 4 min pour la fraction broyée.

### Exemple 2

### 1. Préparation de particules ne contenant pas de tensioactif

Les particules sont préparées selon le mode opératoire décrit à l'exemple 1. La formule de fabrication est indiquée dans le tableau 3.

| Matières premières | Quantité (g) | % |
|---|---|---|
| Lactose EFK | 600 | 40,68 |
| Itraconazole | 350 | 23,73 |
| HMPC 2910 5 cps | 525 | 35,59 |
| Polysorbate 20 | 0 | 0 |
| Alcool 96° | 4 200 | / |
| Chlorure de méthylène | 5 900 | / |
| Quantité de solution pulvérisée | 10 975 | / |
| Masse sèche théorique | 1 475 | / |
| Titre théorique | 237,3 mg/g | / |

### 2. Résultats.

### 2.1. Distribution granulométrique.

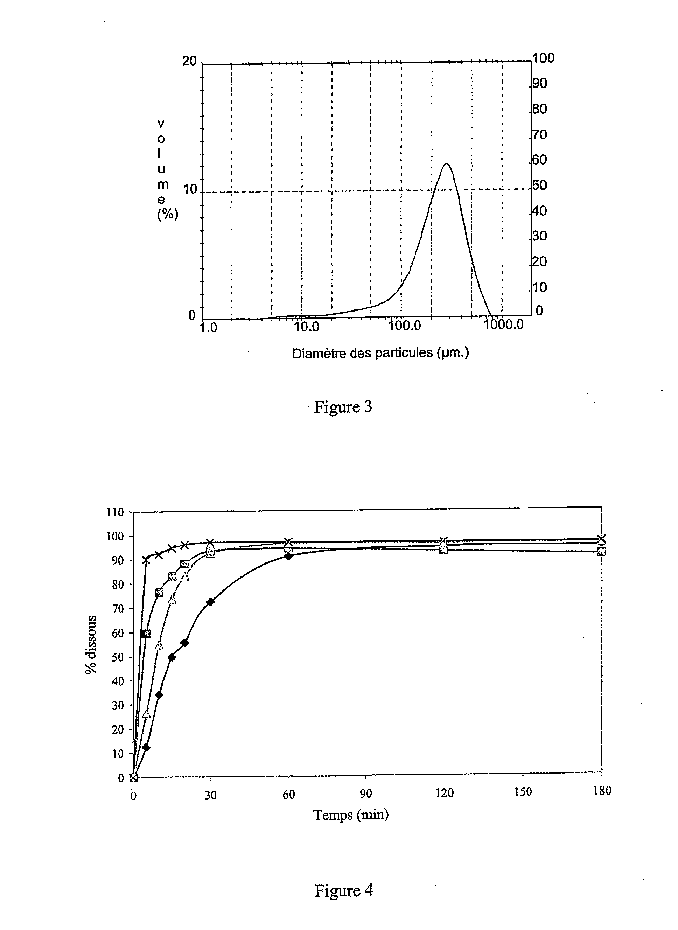

Elle est présentée dans la figure 3. La distribution granulométrique donne les résultats suivants:
- D_{10%} : 88 µm
- D_{50µ} : 239 µm
- D_{90%} : 435 µm.

### 2.2. Cinétique de dissolution.

Elle est étudiée dans les mêmes conditions qu'à l'exemple 1.

Outre le broyage, la présence d'un tensioactif accélère la dissolution. Les résultats sont illustrés dans la figure 4.

Après 10 minutes de dissolution, 26% d'itraconazole est dissous pour le lot sans tensioactif, alors que 90% d'itraconazole est dissous pour le lot avec tensioactif pour lequel pratiquement la totalité de l'itraconazole co-précipité est dissoute.

## Revendications

1. Procédé de préparation d'une particule constituée d'un co-précipité appliqué en couches autour d'un support neutre hydrophile par pulvérisation d'une solution organique sur ledit support neutre hydrophile, ladite solution comprenant au moins une substance active pratiquement insoluble dans l'eau, un agent tensioactif et un polymère hydrophile, **caractérisé en ce qu'**il comprend les étapes suivantes :
a) préparation d'une solution organique comprenant la substance active, le polymère hydrophile et l'agent tensioactif,
b) pulvérisation d'une partie de la solution obtenue en a) sur les supports neutres hydrophiles,
c) broyage des particules obtenues à l'étape b),
d) pulvérisation de la quantité restante de la solution organique sur les particules broyées à l'étape c), et
e) broyage final des particules obtenues à l'étape d).

2. Procédé de préparation des particules selon la revendication 1, **caractérisé en ce que** la séquence pulvérisation/broyage (étapes b à d) est répétée une ou plusieurs fois.

3. Procédé de préparation des particules selon l'une quelconque des revendications 1 et 2, **caractérisé en ce qu'**il comprend en outre une étape de séchage soit après chaque étape de pulvérisation avant broyage, soit immédiatement après le broyage.

4. Procédé de préparation des particules selon l'une quelconque des revendications 1 à 3, **caractérisé en ce que** le support inerte hydrophile est constitué par tout excipient inerte chimiquement et pharmaceutiquement, existant sous forme particulaire, cristalline ou amorphe, et choisi de préférence dans le groupe comprenant des dérivés de sucres, des celluloses ou leurs mélanges.

5. Procédé de préparation des particules selon l'une quelconque des revendications 1 à 4, **caractérisé en ce que** le polymère hydrophile est choisi dans le groupe comprenant les polyvinylpyrrolidones, notamment les polymères de poids moléculaire compris entre 10 000 et 50 000, les dérivés cellulosiques, de préférence l'hydroxypropylméthylcellulose, l'hydroxyproprylcellulose, l'hydroxyméthylcellulose, l'hydroxypropylméthylcellulose phtalate, l'hydroxypropyl méthylcellulose acéto-succinate, les polymères acryliques et les polyéthylènes glycols.

6. Procédé de préparation des particules selon l'une quelconque des revendications 1 à 5, **caractérisé en ce que** l'agent tensioactif est choisi dans le groupe comprenant les agents cationiques, anioniques, non ioniques ou amphotères, seuls ou en mélange.

7. Procédé de préparation des particules selon l'une quelconque des revendications 1 à 6, **caractérisé en ce que** le solvant organique est choisi dans le groupe comprenant l'éthanol, l'isopropanol, le tétrahydrofuranne, l'éther isopropylique, l'acétone, la méthyléthylacétone, le chlorure de méthylène et les mélanges de ces solvants.

8. Procédé de préparation des particules selon l'une quelconque des revendications 1 à 7, **caractérisé en ce que** les étapes de pulvérisation sont réalisées dans une turbine à dragéification, dans une turbine perforée ou en lit fluidisé.

9. Particule constituée d'un co-précipité appliqué en couches autour d'un support neutre hydrophile et comprenant au moins une substance active pratiquement insoluble dans l'eau, un agent tensioactif et un polymère hydrophile, caractérisée ce que qu'elle est susceptible d'être obtenue par le procédé selon l'une quelconque des revendications 1 à 8.

10. Particule selon la revendication 9, **caractérisée en ce que** la substance active est présente dans la particule dans une proportion pouvant varier entre 1 et 60% en poids.

11. Particule selon l'une quelconque des revendications 9 et 10, **caractérisée en ce que** le support inerte hydrophile est présent dans une proportion pouvant aller jusqu'à 95% en poids.

12. Particule selon l'une quelconque des revendications 9 à 11, **caractérisée en ce que** le rapport pondéral polymère hydrophile/principe actif est compris entre 10/1 et 1/2.

13. Particule selon l'une quelconque des revendications 9 à 12, **caractérisée en ce que** l'agent tensioactif est présent dans une proportion pouvant varier entre 0,1 et 20% en poids par rapport à la masse totale obtenue.

14. Particule selon l'une quelconque des revendications 9 à 13, **caractérisée en ce que** la dimension particulaire unitaire du support inerte hydrophile peut être comprise entre 50 et 500µm, de préférence entre 90 et 200µm.

15. Forme pharmaceutique **caractérisée en ce qu'**elle contient au moins une particule selon l'une quelconque des revendications 9 à 14, éventuellement associée à des excipients pharmaceutiquement acceptables.

## Patentansprüche

1. Verfahren zur Herstellung eines Partikels, das von einem Co-Präzipitat gebildet ist, welches in Schichten um einen hydrophilen neutralen Träger herum aufgebracht wird mittels Zerstäuben einer organischen Lösung auf den hydrophilen neutralen Träger, wobei die Lösung mindestens einen in Wasser praktisch unlöslichen Wirkstoff, ein Tensid und ein hydrophiles Polymer enthält, **dadurch gekennzeichnet, dass** es die folgenden Schritte umfasst:
a) Herstellen einer organischen Lösung, die den Wirkstoff, das hydrophile Polymer und das Tensid enthält,
b) Zerstäuben eines Teils der in a) erhaltenen Lösung auf den hydrophilen neutralen Trägern,
c) Zerkleinern der in Schritt b) erhaltenen Partikel,
d) Zerstäuben der Restmenge der organischen Lösung auf die in Schritt c) zerkleinerten Partikel, und
e) abschließendes Zerkleinern der in Schritt d) erhaltenen Partikel.

2. Verfahren zur Herstellung der Partikel nach Anspruch 1, **dadurch gekennzeichnet, dass** die Abfolge Zerstäuben/Zerkleinern (Schritt b bis d) ein oder mehrere Male wiederholt wird.

3. Verfahren zur Herstellung der Partikel nach einem Ansprüche 1 und 2, **dadurch gekennzeichnet, dass** es ferner einen Schritt des Trocknens entweder nach jedem Schritt des Zerstäubens vor dem Zerkleinern, oder unmittelbar nach dem Zerkleinern umfasst.

4. Verfahren zur Herstellung der Partikel nach einem der Ansprüche 1 bis 3, **dadurch gekennzeichnet, dass** der hydrophile inerte Träger von einem beliebigen chemisch und pharmazeutisch inerten Träger gebildet ist, der in Partikelform, kristalliner Form oder amorpher Form vorliegt und bevorzugt ausgewählt ist aus der Gruppe umfassend Zuckerderivate, Zellulosen oder Gemische davon.

5. Verfahren zur Herstellung der Partikel nach einem der Ansprüche 1 bis 4, **dadurch gekennzeichnet, dass** das hydrophile Polymer ausgewählt ist aus der Gruppe umfassend Polyvinylpyrrolidone, insbesondere Polymere mit einem Molekulargewicht zwischen 10.000 und 50.000, Zellulosederivate, bevorzugt Hydroxypropylmethylcellulose, Hydroxypropylcellulose, Hydroxymethyl-cellulose, Hydroxypropylmethylcellulosephthalat, Hydroxypropylmethylcelluloseacetatsuccinat, Acryl-Polymere und Polyethylenglykole.

6. Verfahren zur Herstellung der Partikel nach einem der Ansprüche 1 bis 5, **dadurch gekennzeichnet, dass** das Tensid ausgewählt ist aus der Gruppe umfassend kationische, anionische, nichtionische oder amphotere Mittel, allein oder als Gemisch.

7. Verfahren zur Herstellung der Partikel nach einem der Ansprüche 1 bis 6, **dadurch gekennzeichnet, dass** das organische Lösemittel ausgewählt ist aus der Gruppe umfassend Ethanol, Isopropanol, Tetrahydrofuran, Isopropylether, Aceton, Methylethylaceton, Methylenchlorid und die Gemische dieser Lösemittel.

8. Verfahren zur Herstellung der Partikel nach einem der Ansprüche 1 bis 7, **dadurch gekennzeichnet, dass** die Schritte des Zerstäubens in einer Dragierturbine, in einer perforierten Turbine oder in einer Wirbelschicht durchgeführt werden.

9. Partikel, das von einem Co-Präzipitat gebildet ist, welches in Schichten um einen hydrophilen neutralen Träger herum aufgebracht ist und mindestens einen in Wasser praktisch unlöslichen Wirkstoff, ein Tensid und ein hydrophiles Polymer enthält, **dadurch gekennzeichnet, dass** es durch das Verfahren nach einem der Ansprüche 1 bis 8 erhalten werden kann.

10. Partikel nach Anspruch 9, **dadurch gekennzeichnet, dass** der Wirkstoff in dem Partikel in einem Anteil vorhanden ist, der zwischen 1 und 60 Gew.-% variieren kann.

11. Partikel nach einem der Ansprüche 9 und 10, **dadurch gekennzeichnet, dass** der hydrophile inerte Träger in einem Anteil vorhanden ist, der bis zu 95 Gew.-% betragen kann.

12. Partikel nach einem der Ansprüche 9 bis 11, **dadurch gekennzeichnet, dass** das Gewichtsverhältnis hydrophiler Träger/Wirkstoff zwischen 10/1 und 1/2 beträgt.

13. Partikel nach einem der Ansprüche 9 bis 12, **dadurch gekennzeichnet, dass** das Tensid in einem Anteil vorhanden ist, der zwischen 0,1 und 20 Gew.-% relativ zu der erhaltenen Gesamtmasse variieren kann.

14. Partikel nach einem der Ansprüche 9 bis 13, **dadurch gekennzeichnet, dass** die Einzelteilchengröße des hydrophilen inerten Trägers zwischen 50 und 500 µm, bevorzugt zwischen 90 und 200 µm beträgt.

15. Pharmazeutische Form, **dadurch gekennzeichnet, dass** sie mindestens ein Partikel nach einem der Ansprüche 9 bis 14 enthält, gegebenenfalls in Verbindung mit pharmazeutisch verträglichen Trägern.

## Claims

1. A process for the preparation of a particle composed of a coprecipitate applied in layers around a neutral hydrophilic carrier by spraying an organic solution over said neutral hydrophilic carrier, said solution comprising at least one active substance substantially insoluble in water, one surface-active agent and one hydrophilic polymer, **characterized in that** it comprises the following stages:
a) preparing an organic solution comprising the active substance, the hydrophilic polymer and the surface-active agent,
b) spraying a portion of the solution obtained in a) over the neutral hydrophilic carriers,
c) milling the particles obtained in stage b),
d) spraying the remaining amount of the organic solution over the particles milled in stage c), and
e) final milling of the particles obtained in stage d).

2. The process for the preparation of the particles according to claim 1, **characterized in that** the spraying/milling sequence (stages b to d) is repeated one or more times.

3. The process for the preparation of the particles according to any one of claims 1 and 2, **characterized in that** it further comprises a drying stage either after each spraying stage, before milling, or immediately after the milling.

4. The process for the preparation of the particles according to any one of claims 1 to 3, **characterized in that** the inert hydrophilic carrier is composed of any chemically and pharmaceutically inert excipient existing in the crystalline or amorphous particulate form and preferably chosen from the group consisting of sugar derivatives, celluloses or mixtures thereof.

5. The process for the preparation of the particles according to any one of claims 1 to 4, **characterized in that** the hydrophilic polymer is chosen from the group consisting of polyvinylpyrrolidones, in particular polymers with a molecular weight of between 10 000 and 50 000, cellulose derivatives, preferably hydroxypropylmethylcellulose, hydroxypropylcellulose, hydroxymethyl-cellulose, hydroxypropylmethylcellulose phthalate, hydroxypropylmethylcellulose acetate/succinate, acrylic polymers and polyethylene glycols.

6. The process for the preparation of the particles according to any one of claims 1 to 5, **characterized in that** the surface-active agent is chosen from the group consisting of cationic, anionic, nonionic and amphoteric agents, alone or as a mixture.

7. The process for the preparation of the particles according to any one of claims 1 to 6, **characterized in that** the organic solvent is chosen from the group consisting of ethanol, isopropanol, tetrahydrofuran, isopropyl ether, acetone, methyl ethyl ketone, methylene chloride and the mixtures of these solvents.

8. The process for the preparation of the particles according to any one of claims 1 to 7, **characterized in that** the spraying stages are carried out in a coating pan, in a perforated pan coater or in a fluidized bed.

9. A particle composed of a coprecipitate which is applied in layers around a neutral hydrophilic carrier and which comprises at least one active substance substantially insoluble in water, one surface-active agent and one hydrophilic polymer, **characterized in that** it is capable of being obtained by the process according to any one of claims 1 to 8.

10. The particle according to claim 9, **characterized in that** the active substance is present in the particle in a proportion which can vary between 1 and 60% by weight.

11. The particle according to any one of claims 9 and 10, **characterized in that** the inert hydrophilic carrier is present in a proportion which can range up to 95% by weight.

12. The particle according to any one of claims 9 to 11, **characterized in that** the hydrophilic polymer/active principle ratio by weight is between 10/1 and 1/2.

13. The particle according to any one of claims 9 to 12, **characterized in that** the surface-active agent is present in a proportion which can vary between 0.1 and 20% by weight, with respect to the total weight obtained.

14. The particle according to any one of claims 9 to 13, **characterized in that** the unit particle size of the inert hydrophilic carrier can be between 50 and 500 µm, preferably between 90 and 200 µm.

15. A pharmaceutical form, **characterized in that** it comprises at least one particle according to any one of claims 9 to 14, optionally in combination with pharmaceutically acceptable excipients.
